# EUROPEAN PATENT APPLICATION

(11) **EP 4 282 410 A1**
(43) Date of publication of application: **29.11.2023**
(21) Application number: 22175847.7
(22) Date of filing: 27.05.2022
(51) Int. Cl.: A61K 31/198, A61K 38/12, A61P 9/08, A61P 9/14

(54) **ASSOCIATION OF COLISTIN AND N-ACETALCYSTEINE IN THE TREATMENT OF BACTERIAL INFECTIONS BY P. AERUGINOSA IN A MATURE BIOFILM PHASE OF GROWTH**

(71) Applicant: Zambon S.p.A., 20091 Bresso (MI) (IT)
(72) Inventor: Rossolini, Gian Maria, 53042 Chianciano Terme (SI) (IT); Valzano, Felice, 53100 Siena (SI) (IT); Micieli, Maria, 53100 Siena (SI) (IT); Boncompagni, Selene Rebecca, 52100 Arezzo (AR) (IT); Vailati, Silvia, 20091 Bresso (MI) (IT)
(74) Representative: Merli, Silvia

(57) **Abstract**

The present invention relates to the synergic pharmacological association of colistin and N-acetylcysteine (NAC) for the treatment of bacterial infections caused by the pathogen *P. aeruginosa, wherein said pathogen is in a mature biofilm phase of growth.* The synergic association is in particular useful for the treatment and eradication of *P. aeruginosa* infections in patients suffering from chronic lung diseases and recurrent *P*. *aeruginosa* infections.

## Description

### FIELD OF THE INVENTION

The present invention relates to the synergic combination of two known drugs for the treatment of biofilm-associated *P. aeruginosa* infections.

### STATE OF THE ART

According to National Institutes of Health (NIH) about 65% of all microbial infections, and 80% of all chronic infections are associated with the presence of bacterial biofilms.

However, it has been only recently defined that bacterial biofilms represent a big threat to the public health because they confer pathogens a reduced susceptibility to antibiotics and to the host immune system.

Furthermore, biofilm-associated infections are involved in the worsening of a variety of infectious diseases caused by MDR (Multidrug resistant) bacteria, which very often develop resistance to the more recently developed antibiotics. In fact, very often, the antibiotics currently used may decrease the number of bacteria in biofilms, but they cannot completely eradicate bacteria in this protected environment (Fernandez Barat, 2017 et al. J. Cyst. Fibros. 16, 222-229 DOI:https://doi.org/10.1016/j.jcf.2016.08.005).

The Extracellular Polymeric Substance (EPS) (biofilm in the following) is made of proteins (<1-2%, including enzymes), DNA (<1%), polysaccharides (1-2%), RNA (<1%) and water (up to 97%) that allows the flow of nutrients inside the biofilm matrix.

Biofilm formation is due to the activation of bacterial mechanisms and gene transcription which are not common to the planktonic phase. As a matter of fact, when a bacterial cell switches to the biofilm phase of growth, it undergoes a phenotypic shift in behavior in which large suites of genes are differentially regulated and expressed (Blasi F. et al. Respiratory Medicine, 2016, 117: 190-197; Becker P. et al., Appl. Env. Microbiology, July 2001, 67(7): 2958-2965; Trubenova et al. Trends in microbiol, March 22, 2022, https://doi.org/10.1016/j.tim.2022.02.005).

De facto, biofilm-forming pathogens exhibit features, including resistance to antibiotics, which are different from those exhibited in the planktonic growth phase by the same pathogen. These differences explain why many antibiotics, effective on planktonic - rapidly growing - bacteria, are ineffective against the same, embedded in biofilms, where they also have a reduced growth rate or are quiescent and thus less sensible to the mechanisms behind the most common antibiotic activities.

This may also explain why also diagnostic means may fail in identifying antibiotic susceptibility in bacterial infections and the most suitable antibiotic therapy: diagnostic tests are typically done on bacteria in the planktonic phase of growth, while the infection is maintained in a latent state by biofilm-growing bacteria (Trubenova et al. 2022). Furthermore, biofilm growing bacteria have been studied until recently by standard assays developed for planktonic bacteria, while the study of biofilm growing bacteria in this phase need specific tests which, unfortunately, still lack good standardization.

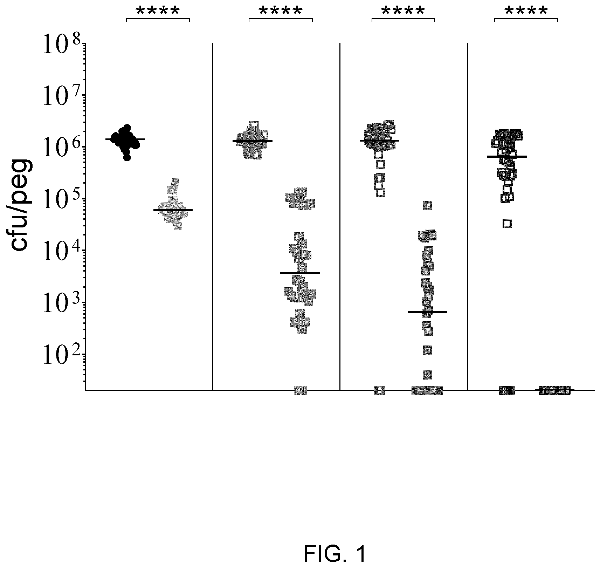

P. aeruginosa has a high incidence among hospital acquired infections and correlates with a high mortality rate. Furthermore, it is involved in several persistent biofilm infections and is a key pathogen in chronic infections of the lung. Morse et al. (Frontiers in Immunology, 2021, 12: 1-15) have reviewed the interactions between this pathogen, in the biofilm phase of growth, and the human immune system and Figure 1 reports a clear schematic of the different maturation stages of biofilms, with young and mature/old biofilms, each one comprising different cell populations as well as different features. Usually, biofilm-associated infections remain asymptomatic until biofilm has reached the mature/old phase and start releasing planktonic cells or biofilm pieces (i.e. the so named septic emboli), which are responsible for the clinical manifestation of the diseases. Conventional antibiotic treatment can often kills the planktonic cells released from the biofilm and part of the biofilm-embedded cells, "persister" cells (quiescent cells not susceptible to antibiotic due to a peculiar physiologic state) are maintained within the biofilm and are ready to promote relapse of infection.

Furthermore, in *P. aeruginosa* the polysaccharidic component, in particular rhamnolipids, has been shown to act as a powerful detergent causing cellular necrosis and neutrophils elimination, thus further shielding the pathogen from the host immune response (Jensen P.Ø et al., Microbiology, 2007, 153: 1329-1338).

In the literature, controversial results have been reported on the supposed interaction between antibiotics and mucolytic agents such as NAC. In 1981, Roberts and Cole found that 2%-5% of NAC exhibited antimicrobial activity against *P. aeruginosa* and that the effect of the carbenicillin on *P. aeruginosa* was augmented by low concentrations of NAC (Journal of Infection Volume 3, Issue 4, December 1981, Pages 353-359).

In 2016, the result of a study carried out by Landini et al. on the effect of high NAC concentrations on antibiotic activity against a collection of respiratory pathogens, demonstrated that high NAC concentrations do not interfere with the activity of the most commonly used antibiotics, whereas NAC compromised the activity of carbapenems (Landini et al. Antimicrob. Agents Chemother. December 2016 vol. 60 no. 12 7513-7517).

However, all the biologic mechanisms elicited by NAC have not yet been fully elucidated.

A strong synergic activity of NAC and colistin has already been described in WO2018/154091, for S. *maltophilia* and *A. baumannii,* by the same Applicant. Infections by these pathogens are usually harmless for healthy people, causing severe pneumonia only in immunocompromised patients, and only seldomly associated with a lethal outcome.

*P. aeruginosa* is involved in several persistent biofilm infections. In chronic lung patients, *P. aeruginosa* infections are associated with frequent exacerbations and higher mortality rates (Malhotra et al. 2019 Clin. Microbiol. Rev. 32(3):e00138-18; Parkins et al. Clin Microbiol Rev 31(4):e00019-18). Furthermore, the activation of the innate and adaptive immunity of the host leads to a chronic inflammatory state and to extensive tissue damages deeply affecting lung functionality. This renders an effective therapy to this pathogen extremely urgent, in particular for these patients.

Of note, in patients affected by cystic fibrosis or other chronic respiratory diseases, colistin is commonly used as a last resort treatment for infections caused by this or other multidrug resistant non-fermenting Gram-negative pathogens (e.g. *Acinetobacter baumannii, Stenotrophomonas maltophilia),* thus also increasing the risk of developing and spreading resistance to this antibiotic among pathogens belonging to different strains.

Therefore, new therapeutic options, able to overcome and bypass the immune-escaping mechanisms of bacterial pathogens, in particular the key pathogen *P. aeruginosa,* are extremely urgent in view of the diffusion of the multidrug resistant phenotype over the last few years and the low efficacy of the new generation antibiotics.

### SUMMARY OF THE INVENTION

The present invention relates to the use of the synergic association of colistin and *N*-acetylcysteine (NAC) for use in the treatment of a bacterial infection caused by the pathogen *P. aeruginosa,* wherein said pathogen is in the biofilm phase of growth. Preferably said bacterial infection is associated with a respiratory disease, such as Chronic Obstructive Pulmonary Disease (COPD), Cystic Fibrosis (CF), Non-Cystic Fibrosis Bronchiectasis (NCFB), Ventilator-Associated Pneumonia (VAP) and exacerbations thereof.

The association is particularly useful on *P. aeruginosa* infections where the pathogen has a Multi-Drug Resistant (MDR) or colistin resistant (CSTR) phenotype, such as for example in hospital associated infections (HAI) or health care-associated infections (HCAI).

The association of colistin and NAC is synergic also when the two drugs are administered concurrently, even not together, in either order, or separately with overlapping or non-overlapping periods of administration, via the same or different administration route. According to a preferred embodiment, colistin is administered by the inhalatory route.

It is a further object of the present invention a kit of parts comprising a vial of colistin in powder and a vial of NAC aqueous solution or as a tablet or granules optionally in combination with dilution means, for the combined administration of the synergic association of colistin and NAC.

Therapeutic methods for the treatment of a *P. aeruginosa* infection with the synergic association of colistin and NAC, wherein the pathogen is in the biofilm phase of growth, as well as therapeutic methods for the eradication of such bacterial infections in a subject in need thereof, are also embodiments comprised within the present invention.

### BRIEF DESCRIPTION OF THE DRAWINGS

**Figure 1****.** Antibiofilm activity of *N*-acetylcysteine (NAC) 8000 mg/L (grey squares), colistin 2, 4 and 8 mg/L (open squares with light grey, dark grey and black borders, respectively), and NAC 8000 mg/L in combination with colistin 2, 4 and 8 mg/L (grey squares with light grey, dark grey and black borders, respectively) against *P. aeruginosa* Z154 from CF (mucoid and colistin-susceptible). Filled circles represent biofilms in absence of NAC and/or colistin (i.e., controls). Black solid lines above symbols represent the median values. The x-axis is set at the limit of detection (i.e., 20 cfu/peg). Statistical significance was determined using Kruskal-Wallis test with Dunn's correction. ****, p<0.0001. cfu: colony forming units.

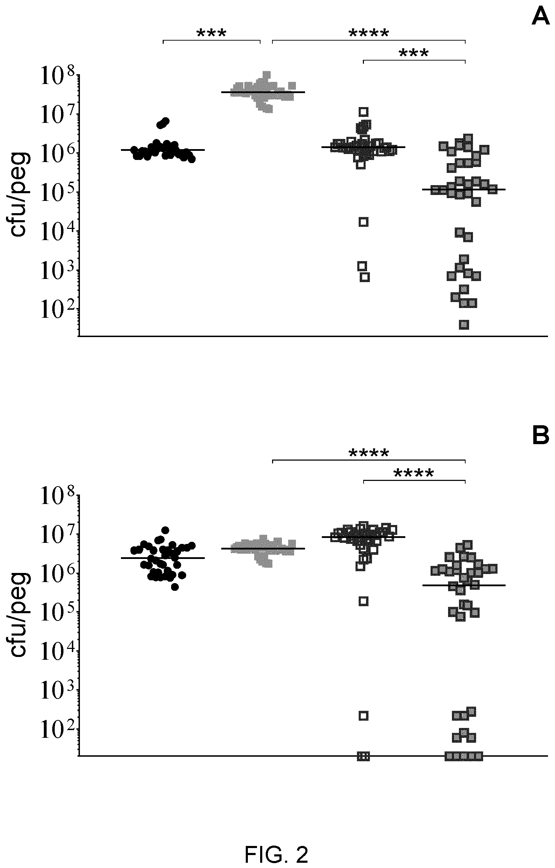

**Figure 2****.** Antibiofilm activity of *N*-acetylcysteine (NAC) 8000 mg/L (grey squares), colistin 8 mg/L (open squares), and NAC/colistin combination (grey squares with black border) against *P. aeruginosa* PAO1 (A) and *P*. *aeruginosa* Z34 from CF (B) (both non-mucoid and colistin-susceptible). Filled circles represent biofilms in absence of NAC and/or colistin (i.e., controls). Black solid lines above symbols represent the median values. The x-axis is set at the limit of detection (i.e., 20 cfu/peg). Statistical significance was determined using Kruskal-Wallis test with Dunn's correction. ***, p<0.001; ****, p<0.0001. cfu: colony forming units.

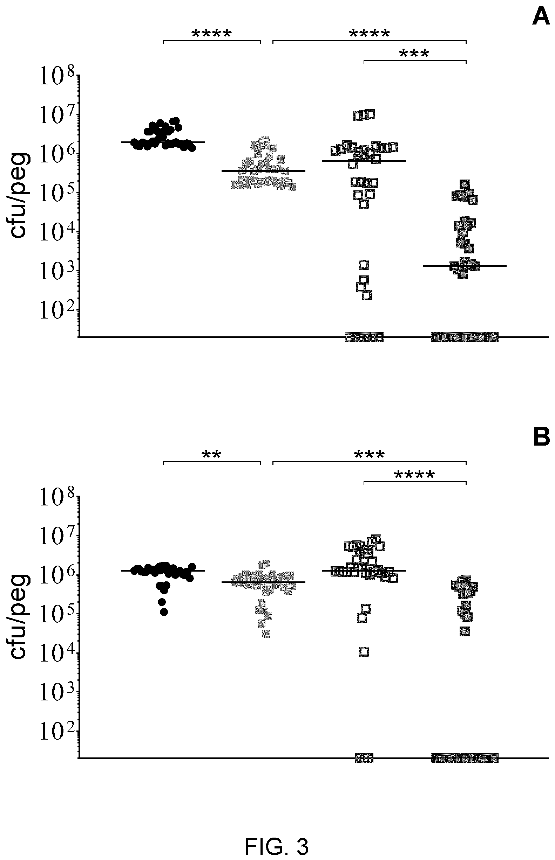

**Figure 3****.** Antibiofilm activity of *N*-acetylcysteine (NAC) 8000 mg/L (grey squares), colistin 8 mg/L (open squares), and NAC/colistin combination (grey squares with black border) against *P. aeruginosa* M19 (A) and *P. aeruginosa* M25 (B) (both from CF, mucoid and colistin-susceptible). Filled circles represent biofilms in absence of NAC and/or colistin (i.e., controls). Black solid lines above symbols represent the median values. The x-axis is set at the limit of detection (i.e., 20 cfu/peg). Statistical significance was determined using Kruskal-Wallis test with Dunn's correction. **, p<0.01; ***, p<0.001; ****, p<0.0001. cfu: colony forming units.

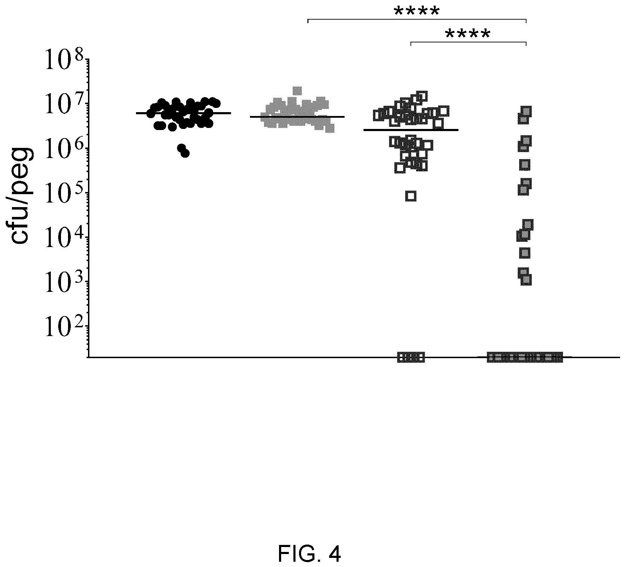

**Figure 4****.** Antibiofilm activity of *N*-acetylcysteine (NAC) 8000 mg/L (grey squares), colistin 32 mg/L (open squares), and NAC/colistin combination (grey squares with black border) against *P. aeruginosa* FC238 from CF (non-mucoid and colistin-resistant). Filled circles represent biofilms in absence of NAC and/or colistin (i.e., controls). Black solid lines above symbols represent the median values. The x-axis is set at the limit of detection (i.e., 20 cfu/peg). Statistical significance was determined using Kruskal-Wallis test with Dunn's correction. ****, p<0.0001. cfu: colony forming units.

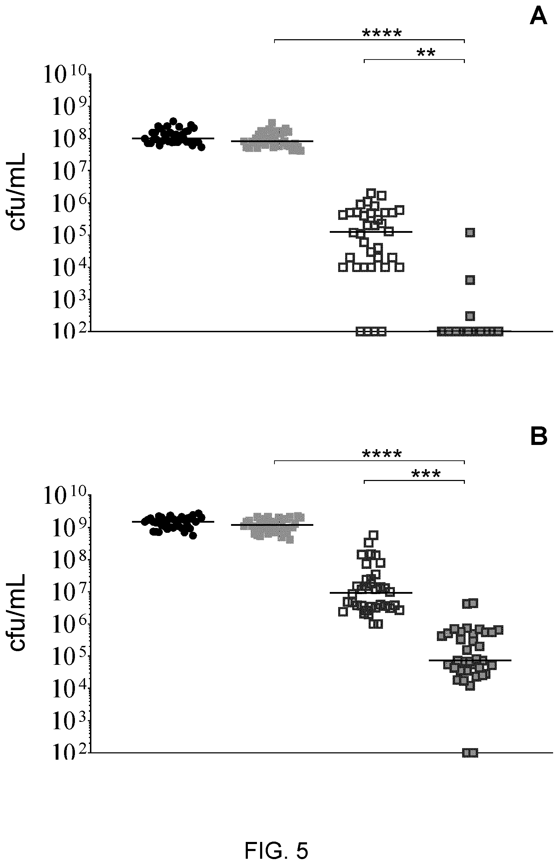

**Figure 5****.** Antibiofilm activity of *N*-acetylcysteine (NAC) 8000 mg/L (grey squares), colistin 64 mg/L (open squares), and NAC/colistin combination (grey squares with black border) against *P. aeruginosa* Z154 (A) and *P. aeruginosa* Z34 (B), in the ASM biofilm model. Filled circles represent biofilms in absence of NAC and/or colistin (i.e., controls). Black solid lines above symbols represent the median values. The x-axis is set at the limit of detection (i.e., 100 cfu/mL). Statistical significance was determined using Kruskal-Wallis test with Dunn's correction. **, p<0.01; ***, p<0.001; ****, p<0.0001. cfu: colony forming units.

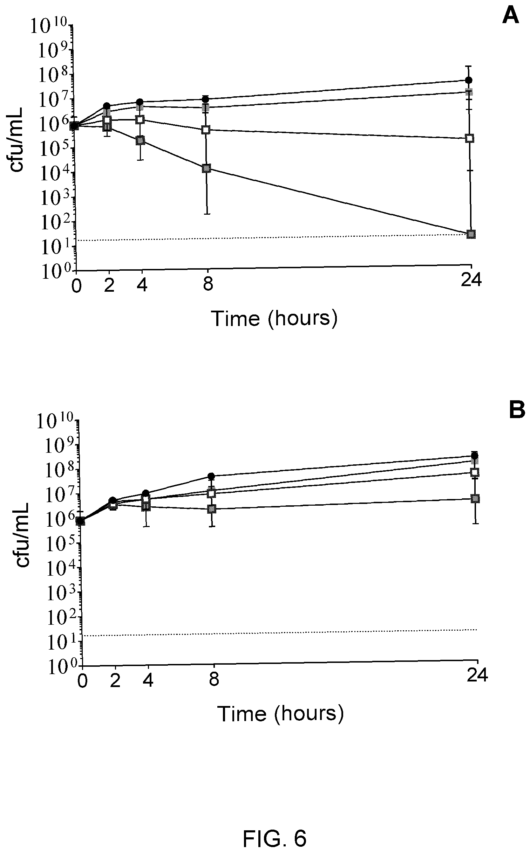

**Figure 6****.** Killing curves demonstrating the differential effect of anaerobic (A) and aerobic (B) conditions on *N*-acetylcysteine (NAC) 8000 mg/L (grey squares), colistin 0.25 mg/L (open squares), and NAC/colistin combination (grey squares with black border) treatment of exponentially growing planktonic cultures of *P. aeruginosa* Z154. Filled circles represent planktonic cultures in absence of NAC and/or colistin (i.e., controls). Median values with range are plotted. Dotted lines indicate the detection limit (i.e., 17 cfu/mL). cfu: colony forming units.

### DETAILED DESCRIPTION OF THE INVENTION

According to the present invention the term "colistin", also termed polymixin E, consists of a cationic cyclic heptapeptide with a tripeptide side chain acylated at the N terminus by a fatty acid through an α-amide linkage. In the following, the term colistin includes colistin and its pharmaceutically acceptable salts and/or prodrugs such as colistin sulfate, colistimethate sodium, colistin methane sulphate and colimycin or colomycin, all intended to refer to the bactericidal cyclopeptide antibiotic colistin or precursors thereof such as the prodrug colimycin which is converted to the active drug colistin.

Two different forms of colistin are available for clinical use: colistin sulfate which is administered orally for bowel decontamination and topically as a powder for the treatment of bacterial skin infections, and colistimethate sodium (CMS) (also called colistin methanesulfate, pentasodium colistimethanesulfate, and colistin sulfonyl methate) for parenteral (intravenous, intramuscular, aerosolized and intrathecal/intraventricular) therapy. Colistin can be therefore administered as a prodrug in form of colistimethate sodium, which is readily hydrolyzed to form sulfomethylated derivatives, as well as colistin sulfate, the active form of the drug. In a particular aspect, for in vitro susceptibility testing in the experimental part of the present invention, the term "colistin" refers to colistin sulfate (in accordance with the international guidelines for antimicrobial susceptibility testing provided by the Clinical and Laboratory Standards Institute - CLSI and the European Committee on Antimicrobial Susceptibility Testing - EUCAST).

Colistin has recently gained a crucial role for the treatment of various types of infections (e.g. pneumonia, bacteremia, urinary tract infections) caused by Gram-negative pathogens expressing a multidrug resistance phenotype (e.g. non-fermenting Gram-negative pathogens, and carbapenem-resistant enterobacteria) and represents one of the last therapeutic options for Gram-negative bacteria, among which *P. aeruginosa.* Therefore, the appearance of colistin-resistant *P. aeruginosa* strains, with increased Minimum Inhibitory Concentrations (MICs) is of great concern. In fact, very high colistin amounts are known to be highly toxic to the host and this limits its use even when colistin would be the only choice.

*N*-acetylcysteine, commonly abbreviated as NAC, is the acetylated precursor of both the amino acid L-cysteine and reduced glutathione (GSH). Historically, it has been used as a mucolytic agent with antioxidant and antiinflammatory properties, in patients who have viscid or thickened airway mucus for a range of chronic respiratory illnesses, including chronic bronchitis, emphysema, COPD and exacerbations, cystic fibrosis, bronchiectasis, as an antidote due to acetaminophen overdose and as a potential treatment of diseases characterized by free radical, oxidant damage. NAC is commercially available under various brand names by different manufacturers throughout the world, for example as Fluimucil^{™} (Zambon Spa).

In the perspective of studying more effective therapeutic options against MDR pathogens, able to avoid the use of extremely high (and toxic) antibiotic concentrations needed to eradicate bacterial infections in biofilms, the synergy between colistin and NAC should deserve great attention, first of all because NAC is not an antibiotic and secondly because this molecule is not toxic, or is toxic only at extremely high concentrations. Therefore, its synergy in combination with colistin, allows the use of lower antibiotic concentrations which are nevertheless effective against bacteria embedded in biofilms even with a MDR phenotype.

It represents a first embodiment of the present invention the use of the synergic association of colistin and NAC for the disruption of *P. aeruginosa* already formed biofilms and the bactericidal activity of *P. aeruginosa* cells already in the biofilm phase of growth, enveloped in mature biofilms.

By "biofilm phase of growth" or "biofilm phase" is meant the maturation phase of the biofilm development. It is characterized by cellular division, production of the extracellular matrix, and finally dispersion of cells. This phase is also identified as the "mature biofilm" phase. In vitro assays such as: the crystal violet staining, metabolic or molecular assays, growth on peg-lids, such as the Calgary Biofilm Device (CBD) on a microtiter plate, determination of the minimum biofilm eradication concentration (MBEC) both described in Harrison JJ et al.( Nat Protoc, 2010; 5: 1236-54), Biofilm Ring Test (BRT), Biofilm susceptibility test, or Optical Imaging on a biopsy, growth of the biofilm bacteria on nutrient plate agar after dispersion of planktonic bacteria, are typically used to distinguish a biofilm associated infection. In vivo, biofilm associated infections are associated with recurrency of infection.

The final phase of mature biofilm development, i.e., disruption and dispersion of the bacteria plays an important role in determining persistence and recurrence of biofilm associated infections in patients with a *P*. *aeruginosa* history of infection, in particular in patients suffering from Cystic Fibrosis and other chronic lung diseases (e.g., chronic obstructive pulmonary disease, and non-CF bronchiectasis). In fact, once established in the CF airways, *P. aeruginosa,* which is the prevalent infection in these patients, develops into chronic infections and generally persists indefinitely, contributing to the decline in pulmonary function, frequent exacerbations, and higher rates of mortality.

The results behind the present invention have provided evidence by suitable and specific assays developed for bacteria in the biofilm phase, that the present association of colistin and NAC has a synergic antibiofilm activity on *P. aeruginosa* enveloped in already formed, mature biofilms, according to the definition and the schematic provided e.g. in Moser et al. Front. Immunol. 12: 625597 (doi: 10.3389/fimmu.2021.625597) (see in particular Figure 1), thus avoiding recurrency of infection.

According to the present invention the term "antibiofilm" means the inhibition or suppression of biofilm persistence or maintenance in bacteria which are already in the biofilm phase. In other words, and without being bound to a specific scientific theory, the observed synergistic antibiofilm activity might be due to different mechanisms, such as an increased penetration of the antibiotic in the Extracellular Polymeric Substances (EPS, e.g. biofilm), a direct synergic inhibitory activity on biofilm formation, an inhibition of the bacterial growth and consequent reduction or maintenance of the biofilm embedding the bacteria, as well to the potentiation of the bactericidal activity of each drug.

Chronic infections caused by *P. aeruginosa* are associated with adaptive changes of the pathogen within the lung of chronic patients, such as CF patients. Conversion to a mucoid phenotype, switching to the biofilm mode of growth, and acquisition of antibiotic resistance, caused by cumulative exposure to antibiotics during treatment and leading to the ineffectiveness of the antibiotic therapy and consequently worse clinical outcomes, are frequently observed.

Therefore, the present synergic association is particularly useful for patients suffering from chronic lung diseases and *P. aeruginosa* infections, against their exacerbations and recurrencies.

More generally, the association according to the present invention may represent the best therapeutic option for the eradication of *P. aeruginosa* infections and reduction of lung decline in the pulmonary functions of patients with chronic pulmonary diseases e.g. suffering from Cystic Fibrosis, Chronic Obstructive Pulmonary Disease, and non-CF bronchiectasis. Also, the present association is useful in Ventilator-Associated Pneumonia (VAP).

In fact, without being bound to a specific scientific theory, it is believed that the ability of the colistin and NAC association according to the present invention, to disrupt *P. aeruginosa* biofilms may increase the pathogen susceptibility to the antibiotic. At the same time the inflammation state, induced by the bacteria and also by the some of the biofilm components, is also mitigated, thus improving lung functions in different ways. Until the present results, to the best of our knowledge, no data were yet available on the *P. aeruginosa* biofilm model.

Thus, the observed synergistic antibiofilm activity according to the present invention may result from different mechanisms, acting concurrently or not, and which have been only partially elucidated, such as an increased penetration of the antibiotic in the Extracellular Polymeric Substances (EPS, e.g. biofilm), a direct synergic inhibitory activity on biofilm formation, or direct inhibition of the bacterial growth.

As a matter of fact, even the mechanisms behind the biological activity of NAC have not been fully elucidated and are probably pleiotropic.

According to the present invention the terms "synergistic" and "synergistically" applied to the effect of colistin and NAC used in association (whether simultaneously or sequentially) refer to a greater antibacterial effect obtained with respect to treatment of the above-identified bacteria by either colistin or NAC alone. This synergistic effect can be clearly seen in the drawings enclosed to the present Application where control treatments (colistin or NAC alone) are always included in the assays. Typically, the effect of colistin and NAC used in association (whether simultaneously or sequentially) is greater than the simple addition of the effects of each agent administered alone, i.e. there is an effect which surpasses expectations based on additive effects. Statistical methods, such as those better detailed in the Experimental Part, are available in the field to identify synergy or a simple additive effect.

The synergic association between colistin and NAC is particularly effective on Multi Drug Resistant (MDR) and colistin resistant (CST^{R}) strains and may consistently reduce colistin MIC also in colistin resistant *P. aeruginosa* strains. This is extremely important for antibiotics toxic at very high doses, such as colistin.

The synergy observed is common to different *P. aeruginosa* isolates and is independent of the mucoid or non-mucoid phenotype of the bacteria, the genotype (e.g. ST-type or O-type) and the antibiotic susceptibility or resistance pattern, as determined by standard methods better detailed in the experimental part. This result is extremely relevant, in view of the great variability of the response among isolates belonging to the same pathogen type, as described in (Aiyer A. et al. Antibiotics 2021, 10, 1176. https://doi.org/10.3390/antibiotics10101176), the great heterogeneity of the population of bacteria embedded in the biofilm environment, and last but not least, to the differences between the biological responses of the planktonic and the biofilm phase of growth even within the same isolate.

It is therefore proposed that the present synergic association of colistin and NAC is particularly useful against recurrent *P. aeruginosa* infections or relapses of an infection (often by MDR isolates), and in the eradication of said infections due to its ability to affect bacteria embedded in mature biofilms.

By "relapse" of infections is meant an infection caused by the same pathogen after a successful therapeutic regimen. By "eradication" is meant clearance of the pathogen and recovery of the patient after a given time.

The present synergic association is also useful on nosocomial or Hospital Acquired Infections (HAI), i.e. infections acquired in a hospital or other healthcare facility. To emphasize both hospital and nonhospital settings, it is sometimes instead called a healthcare-associated infection (HAI or HCAI). Such infections can be acquired in hospitals, nursing homes, rehabilitation facilities, outpatient clinics, or other clinical settings. MDR bacteria, in particular MDR *P. aeruginosa* bacteria are usually responsible for HAI or HCAI and are associated with a high mortality.

Colistin and NAC in the synergic association according to the present invention may be administered in either order, separately or concurrently, with overlapping or non-overlapping periods of administration and via the same or different routes of administration.

For example, colistin and NAC can be concurrently administered in a single dosage or in separate dosages forms in either order, concomitantly or sequentially, with overlapping or non-overlapping periods of administration, via the same or different administration routes.

Thus, the synergic association of colistin and NAC may be administered to the patient in the form of one single or separate pharmaceutical compositions.

The pharmaceutical compositions of the present invention may comprise colistin and NAC together with a carrier suitable for pharmaceutical use, and/or one or more excipients, for the concurrent administration of both colistin and NAC. Alternatively, the pharmaceutical formulations of the invention may comprise NAC together with a carrier suitable for pharmaceutical use, and/or one or more excipients and, separately, colistin formulations may comprise a carrier suitable for pharmaceutical use, and/or one or more excipients. Separate pharmaceutical formulations for sequential or concomitant administration of colistin and NAC can provide the synergic association according to the present invention.

According to the present invention, the term "excipient" comprises any inert substance added to a pharmaceutical composition to further facilitate administration of an active ingredient.

According to the present invention, the term "carrier" comprises any substance suitable as a vehicle for delivering colistin and/or NAC to a suitable in vivo or in vitro site.

The compositions (or formulations) comprising the synergic association of the present invention may be administered by any suitable administration route: oral, parenteral (subcutaneous, intramuscular, intraperitoneal or intravenous) or inhalatory, which is the preferred administration route.

According to the present invention, the terms "oral" or "orally" refer to the introduction into the body by mouth whereby absorption occurs in one or more of the following areas of the body: the mouth, stomach, small intestine, and the small blood vessels of the oral mucosa.

Non-limiting examples of pharmaceutical formulations according to the present invention for oral administration include, for example, tablets, coated tablets, granulates, pills, capsules, liquids, gels, syrups, suspensions, and the like, for oral ingestion by an individual. Suitable carriers for oral administration are well known in the art.

For parenteral administration, pharmaceutical compositions according to the invention may be formulated for example in aqueous solutions such as in physiologically compatible buffers or physiological salt buffer. Formulations for injection may be presented in sterile unit dosage forms, for example, in ampoules, or in multi-dose containers with, optionally, an added preservative.

For administration by inhalation route, pharmaceutical formulations according to the invention may be formulated in solutions, suspensions or dry powders of suitable particle size range and delivered by using conventional means, so that optimal quantities are provided to the patient.

According to a particularly preferred embodiment of the invention, the treatment of the above-identified infections is by the inhalation route. In fact, administration by inhalation, allows reaching high drug concentrations in the Epithelial Lining Fluid (ELF) while minimizing systemic toxicity. In particular, colistin is preferably administered by the inhalation route.

Alternatively, the combination of colistin and NAC can be provided by administering one drug by the inhalation route, i.e. colistin, while the other systemically, i.e. orally or parenterally. Particularly preferred is the drug combination in which colistin is administered via the inhalatory route and NAC is administered (by inhalation, orally or parenterally).

By the inhalatory route, colistin is preferably Colistimethate sodium (CMS) in a sterile saline composition, suitable for administration of an amount of CMS corresponding to at least 20 mg CBA/day by inhalation, nebulization or aerosol spray, and *P. aeruginosa* infections.

The following conversion table is given for Colistin Base Activity (CBA), International Units and amount (mg) ("European Medicines Agency completes review of polymyxin-based medicines", December 16, 2014):

| **Colistimethate sodium (IU)** | **Colistimethate sodium mass (mg)** | **Colistin-base activity (CBA) (mg)** |
|---|---|---|
| **12 500** | 1 | 0.4 |
| **150 000** | 12 | 5 |
| **1 000 000** | 80 | 34 |
| **4 500 000** | 360 | 150 |
| **9 000 000** | 720 | 300 |

Preferably the composition for inhalation is extemporarily prepared at the moment of use from a suitable dose of CMS in powder. Suitable compositions comprise CMS in an amount of from at least 30-35 mg CBA/mL to 60-70 mg CBA/mL and are administered twice a day. According to a preferred embodiment, CMS is prepared in a concentration of at least 30-35 mg CBA, in 1 mL solution suitable for inhalation and about one third (0.3 mL) is delivered by inhalation, though a suitable inhalation device, delivering about 10 mg CBA each inhalation.

The sterile saline aqueous solution typically used for dispersing the colistimethate sodium in powder, preferably comprises sodium chloride (NaCl) in concentration of from 0.4% to 0.9% w/v in sterile water for injection (WFI) or a suitable physiological sterile buffered solution. The preferred final NaCl concentration in the solution for inhalation comprising CMS for use in the treatment according to the present invention, is comprised from 0.4% to 0.5% w/V, even more preferably is 0.45% w/V.

As said above, CMS compositions are preferably prepared extemporaneously, i.e. at the moment of use, or prepared and used within 24 hours, if stored at 2 to 8 °C.

According to one embodiment, the amount of colistin and NAC sufficient to have a synergistic effect on a bacterial infection caused by a biofilm-forming *P. aeruginosa* strain may vary, for example, in view of the physical characteristics of the patient, such as the weight, the severity of the subject's symptoms, the form of the infection, the identity and the bacterial load, or the formulations and the means used for administering the drugs. The specific dose for a given subject is usually set by the judgement of the attending physician.

However, as an example, an effective amount of colistin may be comprised from about 0.075 million units to 12 million units (i.e. from about 6 mg to 960 mg), preferably from about 0.5 million units and 12 million units (i.e. between about 40 mg and 960 mg) to be administered in a single dose or in more repeated doses; the effective amount of NAC may vary from 300 mg/die and 4.800 mg/die, to be administered in a single dose or in more repeated doses.

NAC is preferably administered in a dose of from 600 mg/die and 3000 mg/die, in the pharmaceutical form of tablets, liquid compositions for oral or inhalatory use and/or granules .

The inhalation route is preferred for treating infections of the upper and lower respiratory tract, such as those occurring in patients suffering from Cystic Fibrosis and other chronic lung diseases (e.g. chronic obstructive pulmonary disease, and non-CF bronchiectasis), or in Ventilator-Associated Pneumonia (VAP). This administration route can be selected by determining the active dosage of either NAC and/or colistin useful in the Epithelial Lining Fluid (ELF) after inhalation.

A further object of the present invention is represented by a method of treating a bacterial infection caused by *P. aeruginosa* and exacerbations thereof, in a subject in need thereof, which comprises concurrently administering the association of colistin and NAC, wherein the association has a synergistic activity on bacteria enveloped in already established or mature biofilms.

It is a still further object of the present invention a method of treating a bacterial infection caused by *P. aeruginosa* in a subject in need thereof, which comprises separately administering colistin and NAC, wherein the association of both has a synergistic activity on bacteria enveloped in mature biofilms.

Further embodiments of the present invention are represented by methods for the treatment of *P. aeruginosa* infections in a subject undergoing recurrent *P. aeruginosa* infections, in particular by MDR or ColR isolates. Subjects and specific conditions have been well detailed above.

According to the present invention, the terms "individual", "subject" and "patient" are used interchangeably to refer to a member of mammalian species, preferably a human, which is afflicted with a particular disease, disorder or condition.

Summing up, the synergic association of colistin and *N*-acetylcysteine (NAC) is herein proposed for the treatment and eradication of "difficult to treat" biofilm-associated infections by *P. aeruginosa,* more preferably, for the treatment and eradication of *P. aeruginosa* infections in patients suffering from chronic lung diseases and recurrent *P. aeruginosa* infections.

The present invention will be better detailed in the following Experimental Part, where, notably, results have been obtained with *P. aeruginosa* clinical isolates from different types of infections and tested in assays specific to detect activity on biofilm embedded cells. At variance, many if not all prior art studies, were carried out on the reference *P. aeruginosa* strain (PAO1), and mostly in planktonic culture conditions. In fact, methods for studying the antibiotic susceptibility of bacteria embedded in a biofilm have been only recently adopted and standardized. They are based on resting cultures grown in static conditions.

### EXPERIMENTAL PART

### Example 1. Characterization of the P. aeruginosa strains

Fifteen clinical isolates were from CF, a MDR clinical isolate from Respiratory Tract Infection (RTI) from an Intensive Care Unit (ICU), and the reference strain P. aeruginosa PAO1 (Table 1). Identification was performed by MALDI-TOF MS (Bruker, Shimadzu). Antimicrobial susceptibility was determined using the reference broth microdilution method (CLSIMethods2018). Whole genome sequencing of clinical isolates was performed with the Illumina (San Diego, CA, USA) MiSeq platform, using a 2 × 150 bp paired-end approach. Raw reads were assembled using SPAdes (Bankevich et al. 2012 J Comput Biol 19(5):455-77.), and draft genomes were used to determine MLST- and O-types at the Oxford PubMLST site (https://pubmlst.org/) (Jolley et al. 2018 Wellcome Open Res 3:124) and at the Center for Genomic Epidemiology site (https://cge.cbs.dtu.dk/services/PAst-1.0/) (ThraneJCM2016), respectively. The complete genome of P. aeruginosa Z154 was obtained by combining results from Illumina with those obtained using the Oxford Nanopore Technologies (Oxford, UK) MiniON platform, and de novo assembly was generated using Unicycler v0.4.4.

The features of the P. aeruginosa isolates in this study are summarized in Table 1.

PAO1 represents a reference strain (Stover CK et al., Nature, 2000; 406: 959-976)

**Table 1. Characterization of P. aeruginosa strains**

| **Strain** | **Year of isolation** | **Phenotype** | **Origin^{a}** | **ST-type^{b}** | **O-type** | **Resistance pattern^{c}** | **MIC (mg/L)** | |
|---|---|---|---|---|---|---|---|---|
| | | | | | | | **CST** | **NAC** |
| PAO1 | 1954 | Non-mucoid | Wound | ST549 | 05 | Non-resistant pattern | 2 | > 64000 |
| Z33 | 2005 | Non-mucoid | CF | ST235 | O11 | CP^{R}, FQ^{R}, AG^{R} | 1 | 16000 |
| Z34 | 2006 | Non-mucoid | CF | ST17 | O1 | CB^{R}, CP^{R}, FQ^{R}, AG^{R} | 2 | > 16000 |
| Z35 | 2006 | Non-mucoid | CF | ST235 | O11 | Non-resistant pattern | 1 | 16000 |
| Z152 | 2013 | Mucoid | CF | ST155 | 06 | CB^{R}, FQ^{R}, AG^{R} | 2 | 8000 |
| Z154 | 2016 | Mucoid | CF | ST412 | 06 | CP^{R}, FQ^{R}, AG^{R} | 2 | 16000 |
| M1 | 2002 | Mucoid | CF | ST155 | 06 | CB^{R}, CP^{R}, FQ^{R}, AG^{R} | 2 | 16000 |
| M4 | 2005 | Mucoid | CF | ST155 | 06 | CB^{R}, CP^{R}, FQ^{R}, AG^{R} | 2 | 32000 |
| M7 | 2005 | Mucoid | CF | ST253 | O10 | AG^{R} | 2 | 64000 |
| M13 | 2000 | Mucoid | CF | ST274 | 03 | CB^{R}, CP^{R}, AG^{R} | 1 | 32000 |
| M19 | 2006 | Mucoid | CF | ST3509 | 07 | Non-resistant pattern | 1 | 64000 |
| M25 | 2002 | Mucoid | CF | ST235 | O11 | Non-resistant pattern | 2 | 16000 |
| M32 | 2006 | Mucoid | CF | ST235 | O11 | Non-resistant pattern | 2 | 16000 |
| M42 | 2007 | Mucoid | CF | ST2437 | 06 | CB^{R}, CP^{R}, FQ^{R}, AG^{R} | 2 | > 32000 |
| FC237 | 2007 | Non-mucoid | CF | ST365 | 03 | CB^{R}, FQ^{R}, AG^{R}, CST^{R} | 512 | > 32000 |
| FC238 | 2007 | Non-mucoid | CF | ST910 | 06 | CB^{R}, CST^{R} | 8 | > 32000 |
| FZ99 | 2018 | Non-mucoid | RTI_{ICU} | ST111 | 012 | CB^{R}, CP^{R}, FQ^{R}, AG^{R}, CST^{R} | 4 | 64000 |

| | | | | | | | | |
|---|---|---|---|---|---|---|---|---|
| CST, colistin; NAC, *N*-acetylcysteine. ^{a} CF, cystic fibrosis, RTI_{ICU}, respiratory tract infection in intensive care unit (ICU). ^{b} According to MLST Pasteur scheme. ^{c} CB^{R}, resistance to carbapenems (imipenem and meropenem); CP^{R}, resistance to cephems (ceftazidime and cefepime); FQ^{R}, resistance to fluoroquinolones (ciprofloxacin); AG^{R}, resistance to aminoglycosides (amikacin, gentamicin), CST^{R}, resistance to colistin. | | | | | | | | |

### Example 2. Antibiofilm activity of NAC and colistin.

In the present invention the Calgary biofilm device (also referred to as Nunc-TSP lid system in the experimental part) was the method selected to test the *in vitro* antibiofilm activity of colistin and NAC, alone or in combination. NAC stock solutions (100 g/L) were prepared immediately before use. NAC powder (Zambon, Bresso, Italy) was dissolved in sterile distilled water, pH was adjusted at 6.5-6.8 with NaOH 10 M and the solution was filtered through a 0.22-µm membrane filter. All experiments were performed in cation-adjusted Mueller-Hinton broth (CAMHB) (Becton Dickinson, Milan, Italy), unless otherwise specified, starting from an appropriately concentrated medium to avoid broth dilution when NAC solution were used.

**Colistin** Colistin stock solutions (10 mg/L) were prepared immediately before use, by dissolving colistin powder (colistin sulfate, AppliChem, Darmstadt, Germany) in sterile distilled water.

**Assay.** Biofilm susceptibility testing was first performed using the Nunc-TSP lid system (Thermo Fisher Scientific, Waltham, MA, USA), as described previously (Harrison JJ et al., Nat Protoc, 2010; 5: 1236-54). Briefly, biofilms were grown for 24 hours in CAMHB at 35°C under static conditions. Preformed biofilms were then exposed to NAC 8000 mg/L and colistin (colistin sulfate, Applichem, Darmstadt, German) 2 to 32 mg/L, alone and in combination. The colistin concentration was selected according to preliminary results of antibiofilm susceptibility testing (performed by using the above reported biofilm assay) and the colistin MIC for each strain (for colistin-susceptible strains colistin MIC values were 1-2 mg/L; for colistin-resistant strains MIC values were between 4 and 512 mg/L). After 24 hours of exposure (i.e., 35°C, static conditions), biofilms were washed twice with 200 µL of phosphate-buffered saline (PBS) (Sigma Aldrich, Milan, Italy) to remove loosely adherent bacteria, and sessile cells were removed from pegs by sonication for 30 minutes (Elma Transsonic T 460, Singen, Germany) in 200 µL of tryptic soy broth (TSB) (Oxoid, Milan, Italy) supplemented with 1% Tween 20 (Sigma Aldrich) (i.e., the recovery medium). Median cfu per peg (cfu/peg) was then determined by plating 10 µL of appropriate dilutions of the recovery medium onto tryptic soy agar (TSA) (Oxoid) and incubating for 24 hours at 35°C (detection limit, 20 cfu/peg). Colony count was also double checked after 48 hours of incubation. Data from this biofilm model were obtained in at least three independent experiments, with at least twelve replicates per condition per experiment. Statistical analysis of biofilm susceptibility assays was performed using GraphPad Prism version 8.0 (San Diego, CA, USA). Multiple comparison tests were performed by Kruskal-Wallis test with Dunn's correction. RNA-seq statistical analysis was performed using SeqMan NGen v17.3 software tool. **Results.** *P. aeruginosa* Z154 (a mucoid, MDR, colistin-susceptible isolate from CF) was first used for testing the potential antibiofilm synergism of NAC 8000 mg/L plus diverse colistin concentrations. As shown on Figure 1, a relevant synergism was observed already with colistin 2 mg/L (i.e., the colistin MIC for the tested isolate), with a dose-dependent effect at increasing colistin concentrations, and complete biofilm eradication with the combination NAC 8000 mg/L plus colistin 8 mg/L (Fig. 1).

The remaining sixteen isolates were initially tested with the combination NAC 8000 mg/L plus colistin 8 mg/L. In order to detect synergism, the concentration of colistin was modified for bacteria forming biofilms highly susceptible to colistin (n = 7) or particularly resistant (n = 2) (Fig. 2 to 4). Overall, a relevant synergism of NAC/colistin combinations was observed with all tested isolates (including the three colistin-resistant ones). Of note, isolate (FC237) had a very high MIC for colistin, which was reduced to 1/64 in the presence of NAC.

Overall, these data demonstrated that NAC potentiates colistin activity against preformed biofilms of colistin-susceptible and colistin-resistant P. *aeruginosa* isolates from CF, regardless of the mucoid/non-mucoid phenotype, the resistance pattern, and the ST- and O-type. Present findings are consistent with the previously observed antibiofilm synergism of NAC/colistin combinations against colistin-susceptible and colistin-resistant strains of *A. baumannii* and S. *maltophilia* (Pollini et al. 2018 J Antimicrob Chemother, 73: 2388-2395, Ciacci et al. 2019, Antibiotics 8:101).

### Example 3. Activity of NAC/colistin combinations in the Artificial Sputum Medium (ASM) biofilm model

**Methods.** The potential antibiofilm synergism of NAC/colistin combinations was further investigated using an *in vitro* ASM biofilm model (Kirchner et al. 2012 Immunology 64:e3857), in order to mimic *P. aeruginosa* biofilm conditions within the CF mucus. The study was carried out with two selected strains from CF (P. *aeruginosa* Z154 and Z34), exhibiting different features (i.e., mucoid/non-mucoid phenotype, antimicrobial susceptibility pattern, MLST-type, O-type) (Table 1). In brief, biofilms were grown in ASM in 24-well tissue culture treated plates, for 72 hours at 35°C under static conditions. Preformed biofilms were then exposed to NAC 8000 mg/L and colistin 64 mg/L, alone and in combination. Preliminary experiments carried out with lower colistin concentrations (i.e., 2 to 32 mg/L) did not show evident synergistic antibiofilm activity, while higher colistin concentrations (i.e., >64 mg/L) led to eradication of the biofilm cultures even in absence of NAC (data not shown). After 24 hours of exposure (i.e., 35°C, static conditions), bacterial biofilms were disrupted by 30 minutes of sonication followed by manual pipetting, and the median cfu per mL (cfu/mL) was determined following the same protocol described for the Nunc-TSP lid assay. Data from this biofilm model were obtained in at least three independent experiments, with at least twelve replicates per condition per experiment.

**Results.** Two *P. aeruginosa* strains from CF, exhibiting different phenotypes, were selected for susceptibility assays with the ASM biofilm model: P. *aeruginosa* Z34 (non-mucoid, MDR, ST17, O1), and *P. aeruginosa* Z154 (mucoid, MDR, ST412, O6). Biofilms were grown in ASM, in order to mimic the *P. aeruginosa* biofilm environmental conditions experienced in the CF mucus. Preformed biofilms were then challenged in the same medium with NAC/colistin combinations.

As shown in Figure 5, a clear synergism of NAC 8000 mg/L in combination with colistin 64 mg/L was observed with both strains (Fig. 5). Compared to the experiments performed with the Nunc-TSP lid system, the concentration of colistin which allowed to observe a synergism was much higher (i.e., 32 × MIC), possibly due to colistin strong ionic interactions with ASM components (e.g., extracellular DNA, mucin) (Huang et al. 2015 Antimicr Agents Chemioter, 59:5925-5931.). Indeed, preliminary experiments carried out with lower colistin concentrations did not show either colistin antibiofilm activity or synergism with NAC (data not shown). In addition, the antibiofilm activity of NAC alone observed against *P. aeruginosa* Z154 in the Nunc-TSP lid system was not observed in the ASM model (Fig. 5), confirming that the efficacy of NAC alone against preformed *P. aeruginosa* biofilms could be limited *in vivo.*

Overall, these data demonstrated that the antibiofilm synergism of NAC/colistin combinations against *P. aeruginosa* strains from CF is preserved also in the environmental conditions mimicking the CF mucus. This data is particularly promising for clinical applications.

### Example 4. Time-kill assays of NAC/colistin combination against planktonic cultures grown under aerobic and anaerobic conditions.

Time-kill of NAC/colistin assays combinations were performed with P. *aeruginosa* Z154 planktonic culture, both in aerobic and anaerobic conditions.

**Methods.** Time-kill assays were performed according to CLSI guidelines (CLSI Methods M26A) with the colistin-susceptible strain Z154. Colistin 0.25 mg/L was tested alone and in combination with NAC 8000 mg/L under both aerobic and anaerobic conditions. We decided to use this colistin concentration since a higher concentration led to eradication of the planktonic cultures (data not shown). The medium (CAMHB) used to obtain anoxic cultures was placed under anaerobic atmosphere by using AnaeroGen kit (Oxoid) for 3 days prior to use and during the whole experiment. The killing curves were carried out in borosilicate glass bottles with a final volume of 20 mL of CAMHB. At 0, 2, 4, 8 and 24 hours of exposure ten-fold dilutions were plated on TSA, and the number of cfu/mL was determined (detection limit, 17 cfu/mL). Data were obtained from at least four independent experiments, with two replicates per condition per experiment.

**Results.** The results showed a clear bactericidal effect of colistin 0.25 mg/L (1/8 MIC) in combination with NAC 8000 mg/L in planktonic cultures grown in anaerobic conditions (Fig. 6). In particular, eradication of the starting inoculum was achieved after 24 hours of growth. On the other hand, the same cultures grown in the presence of oxygen were not affected by the activity of the combination tested across all of the experiments, demonstrating the influence of growth conditions on susceptibility of P. *aeruginosa* to NAC/colistin combination (Fig. 6). As reported in previous studies, the anaerobic cultures were more susceptible to killing by colistin compared to aerobic cultures and this may be particularly relevant in P. *aeruginosa* biofilm in the CF mucus, where biofilm cells growth under anoxic conditions which are not only related to the bacteria position within the biofilm (i.e., anoxic conditions in the deeper layers), but also by the intense O₂ depletion caused by polymorphonuclear leukocytes (PMNs), determining entire biofilm growing without aerobic respiration. Overall, these results showed a synergism between NAC and colistin in planktonic cultures of a colistin-susceptible *P. aeruginosa* strain grown in absence of oxygen. This is also the first evidence of such a synergism, which was not evident in the same cultures grown in the presence of oxygen. Taken together, these data would support the synergism of NAC/colistin combination observed in the mature biofilms, where most bacterial cells are embedded in an anaerobic niche.

## Claims

1. A synergistic pharmacological association of colistin and N-acetylcysteine (NAC) for use in the treatment of a bacterial infection caused by the pathogen *P. aeruginosa,* wherein said pathogen is in a mature biofilm phase of growth.

2. The association for use according to claim 1, wherein said bacterial infection is associated with a respiratory disease.

3. The association for use according to claim 2, wherein said respiratory disease is a chronic respiratory disease selected from the group consisting of: Chronic Obstructive Pulmonary Disease (COPD), Cystic fibrosis (CF), Non-Cystic Fibrosis Bronchiectasis (NCFB) and exacerbations thereof.

4. The association for use according to claim 2 wherein said respiratory disease is a Ventilator-Associated Pneumonia (VAP).

5. The association for use according to any one of claims 1-4, wherein said *P. aeruginosa* pathogen has at least one of the following features: Multi-Drug Resistant (MDR) or colistin resistant (CST^{R}) phenotype.

6. The association for use according to claim 1, wherein said bacterial infection is a recurrent infection.

7. The association for use according to any one of claims 1-6, wherein said bacterial infection is a hospital associated infection (HAI) or health care-associated infection (HCAI).

8. The association for use according to any one of claims 1-7, wherein colistin and NAC are administered in either order, separately or concurrently, with overlapping or non-overlapping periods of administration, via the same or different administration route.

9. The association for use according to claim 8, wherein colistin and NAC are administered by the inhalatory route.

10. A kit of parts comprising a vial of colistin in powder and NAC is in powder, tablet or aqueous composition, optionally in combination with dilution means, for the combined administration of the synergic association of colistin and NAC.

11. The kit of parts according to claim 10 wherein colistin is diluted in an aqueous composition for administration via the inhalation route.

12. The synergistic pharmacological association of colistin and N-acetylcysteine (NAC) for use according to claim 1 wherein said treatment comprises the eradication of a bacterial infection caused by the pathogen *P. aeruginosa,* and wherein said pathogen is in the mature biofilm phase of growth.

13. Use of the synergistic pharmacological association of colistin and NAC for the preparation of an antibiofilm medicament for the treatment of Gram-negative infections by *P. aeruginosa,* wherein said pathogen is in the mature biofilm phase of growth.

14. The use according to claim 13 wherein said treatment comprises the eradication of the bacterial infection caused by *P. aeruginosa,* wherein said pathogen is in the mature biofilm phase of growth.
